# EUROPEAN PATENT SPECIFICATION

(11) **EP 1 380 255 B1**
(45) Date of publication and mention of the grant of the patent: **02.11.2005**
(21) Application number: 01976948.8
(22) Date of filing: 18.07.2001
(51) Int. Cl.: A61B 5/04, A61B 5/05, A61B 5/053

(54) **ELECTROMAMMOGRAPH**
ELEKTROMAMMOGRAPH
ELECTROMAMMOGRAPHE

(30) Priority: 02.07.2001 RU 2001117743
(43) Date of publication of application: 14.01.2004
(73) Proprietor: Dzhmukhadze, Revaz Longinozovich, Moscow, 119296 (RU); Radina, Elena Vladimirovna, Moscow, 119296 (RU)
(72) Inventor: Dzhmukhadze, Revaz Longinozovich, Moscow, 119296 (RU); Radina, Elena Vladimirovna, Moscow, 119296 (RU)
(74) Representative: Bugnion Genève
(86) International application number: PCT/RU2001/000294
(87) International publication number: WO 2003/003917

(56) References cited:
- WO-A1-00/53090
- RU-C1- 2 116 748
- RU-C1- 2 153 285

## Description

### Field of art

The invention relates to the art of medicine for examination of the population with the aim of detection of the pathologic states of organs and tissues and more specifically to the apparatus for examination of the mammary glands at early stages of the disease. The electric mammograph according to claim 1 is intended for prompt examination (screening) by the electric mammography method.

### Background art

In the prior art, the problem of detection of the pathologic states of organs and tissues, and particularly of the mammary glands, was solved with the aid of a complex stationary equipment which was attended by a specially trained skilled personnel so that the examination could be made only in the diagnostic centres which could be hardly visited by the patients residing and working in the remote districts.

In particular, the prior art comprises the apparatus for detection of the pathologic states of the mammary glands under German patent DE 3017168 which consists of electrodes coupled with the respective inputs of the differential amplifier whose output is coupled with the filter, analog-to-digital converter, interface, and computer This apparatus is deficient in that it is bulky and complicated, its servicing is difficult, the equipment cannot be tuned up and operated in the demonstration and training modes.

The closest Russian analog of the claimed invention is the apparatus under Russian patent RU 2116748 (equivalent to WO 00/53090). This apparatus is also intended for detection of pathologic states of the mammary glands, it has been designed to simplify the diagnostic procedure aid to improve the authenticity and reliability of the results. The apparatus comprises electrodes, a differential amplifier, filter, analog-to-digital converter, optron decoupler, and personal computer for collection and storage of the information on each measurement, calculation of the mathematical expectations and comparison of the absolute values with the threshold values, which characterize the state of the mammary glands. However, this apparatus is also bulky; it should be attended by specially training skilled personnel, it could not be independently used by patients at home. It is unfit for training of the medical personnel and patients, there are no tuning-up devices the electric power is supplied either from the mains or from the cells which should be regularly replaced.

The WO 00/53090 discloses an electric mammograph, which is an electrical impedance tomograph device for obtaining three-dimensional images of the conductivity distribution in biological tissues. A planar array of electrodes (1) is put in contact with the tissues of interest. An AC source (4) is connected to a single remotely attached electrode (5) and to one of the planar array electrodes (1). Potential differences between the electrodes in the planar array and a second remote at rest electrode (7) are measured. The images are reconstructed using a modified back projection method. The electric mammograph device comprises a system of electrodes, said system being connected to an input of a processing unit, a power supply unit, a microcontroller, said processing unit comprising connected in series a filter and analog-to-digital converter, said analog-to-digital converter being connected by a group of inputs-outouts to the microcontroller.

Thus, it was necessary to develop an apparatus without the above-mentioned disadvantages, which is fit for making authentic early diagnostics, can preferably used at home, allows for simultaneous training of the personnel or user, which is cheap, compact, small-sized

### Disclosure of invention

It is an object of the invention to solve the problems associated with elimination of the above-mentioned disadvantages of the prior art apparatus for early diagnostics of mammary gland pathology.

The technical merit achieved through the use of the present invention consists in increase of the authenticity of the early diagnostics, training of the patients with the use of the diagnostic apparatus proper, simplification of the diagnostic procedure.

The electric mammography according to the present invention is defined in claim 1.

Additional technical merit consists in convenient use of the apparatus due to universality of the power supply unit, which comprises an automated storage battery charging device.

Additional technical merit consists in visual display of the examination results.

An additional technical merit is achieved by provision of the power supply unit which comprises connected in series the charging device and the step-up pulse converter connected to the inputs of the microcontroller, while the storage battery is coupled with the analog-to-digital converter.

Another technical merit is achieved by provision of a two-line sign-synthesizing liquid-crystal indicator with the light-emitting diode.

Additionally, the electric mammograph can function in the demonstration mode.

Additionally, the microcontroller is coupled by a group of the inputs-outputs to its own keyboard for selection of the operating modes and for control of the apparatus as a whole.

### Brief description of drawings

The subject-matter of the indention will become more fully apparent from the subsequent description and drawings wherein:
Fig. 1 is a block-diagram of the electronic processing unit with connections,

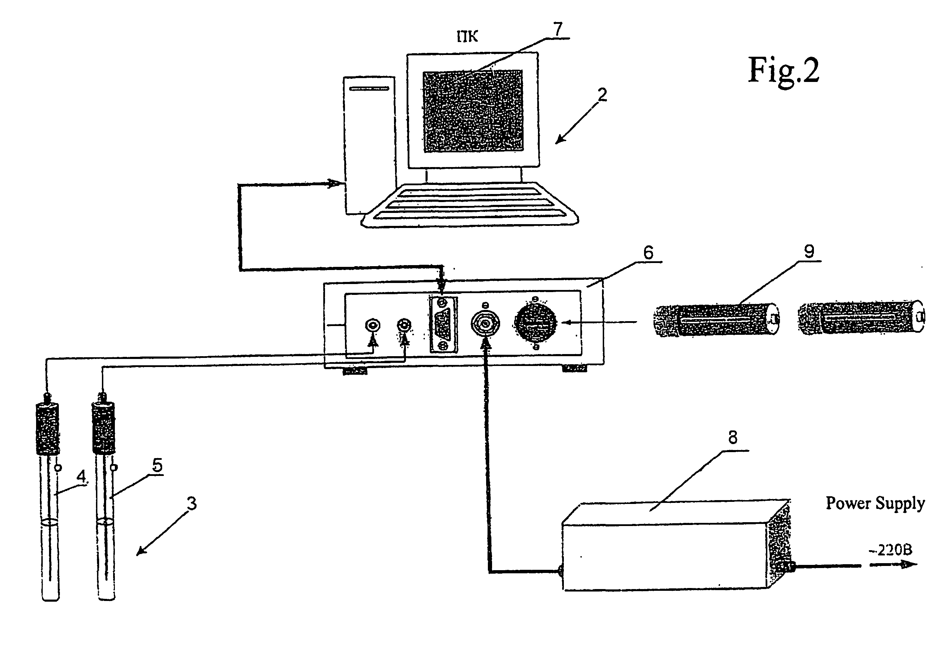

Fig 2 is the diagram snowing connection of the electric mammograph components to the external instruments, and
Fig. 3 shows the layout of the control and indicating organs.

### Best embodiment of invention

The electric mammograph 1 is designed either for joint operation with a personal computer (PC) 2 which provides for processing and display of the obtained information as well as its record for long-term storage with the goal of data bank formation or for operation without the computer

The operation of the electric mammograph 1 is based on measurement in a definite sequence of the difference of the biopotentials in the biologically active points of the patients.

The biopotentials are picked up in biologically active points with the use of a system 3 of electrodes comprising comparison electrodes 4, 5, type 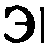 BJI-1M3.1, or similar electrodes. The obtained difference of the biopotentials (signal) is fed to the input 25 of a processing unit 6.

The processing unit 6 filters the signal, matches it own in out resistance with the internal resistance of the signal source, converts the analog signal into the digital form, displays the measurement results on the built-in indicator 11, and transmits them to the personal computer 2 for further processing.

The personal computer 2 processes the obtained data, displays them on a monitor 7, and forms the bank of data for long storage of the accumulated information. The processing unit 6 is supplied with power from a stabilized power supply unit 8 and (or) from a storage battery 9.

The modes of operation of the electric mammograph 1 are determined and selected by the control organs, located on a keyboard 10. The following modes of operation can be selected.

The "Operation" mode which serves for measurement of the potential difference supplied to the input of the processing unit 6. The measurement results are displayed on an indicator 11 of the processing unit 6 and are transmitted through the successive interface to the personal computer 2. The "Operation" mode is evidenced by illumination of the "Operation" inscription on the indicator 11 of the processing unit 6.

The "Control" mode. When this mode is selected, the input of the processing unit 6 is automatically shorted and the input bias voltage of the apparatus, i.e. its initial unbalance, is measured. The "Control" mode is intended for initial balancing of the processing unit. Its selection is evidenced by illumination of the "Control" inscription on the indicator 11.

The "Demonstration" mode is selected by depression of the < or > buttons. In this case, the input measuring part of the processing unit 6 does not function. After one of these buttons is repeatedly depressed or it is depressed and held in position, the processing unit 6 generates the pseudopotentials in the range of ±100 mV at the increments of 1 mV which are displayed on the indicator 11 of the processing unit 6 and are simultaneously transmitted through the successive interface to the personal computer 2.

The "Demonstration" mode is evidenced by the illumination of the "Demonstration" inscription on the indicator 11 of the processing unit 6. The "Demonstration" mode is switched off by the depression of the multi-functional button marked "Balance". When this is done, the processing unit 6 is changed over into the "Operation" mode.

The "Demonstration" mode is intended for training medical personnel and for acquisition of practical skills by the independent user.

In general case, the balancing consists in automatic "zeroing" of the measurement range scale on the processing unit 6, i.e. the input voltage is taken as the zero of the scale.

The balancing of the processing unit 6 is subdivided into the initial balancing and system balancing.

The initial balancing is selected by depression of the "Balance" button in the "Control" mode, i.e. when the input of the processing unit 6 is shorted, and is intended for compensation of its internal bias voltage. The initial balancing is connected or, if necessary, by depression of the "Balance" button in the process of operation.

The system balancing is selected by depression of the "Balance" button in the "Operation" mode, with the electrodes connected and dipped into the working solution. It is intended to compensate for the system unbalance, i.e. inequality of the potentials of the pair of the electrodes which are used.

The system balancing is carried out before taking measurements, when the electrodes 4, 5 (electrode) are replaced, in case of change ("error") of the potential of the electrodes 4, 5, and in other cases which call for compensation of the unbalance of the system 3 of electrodes.

There are three versions of power supply to the processing unit 6:
1. The stabilized power supply unit 8 is connected to the processing unit 6 fitted with the storage battery 9.
   When the stabilized power supply unit 8 is connected to the electric mains, the power is supplied to the processing unit 6 and the battery 9 is charged at a high rate. The time of high-rate charging of the discharged and serviceable battery 9 is not over 5 hours. After termination of high-rate charging, the battery pulse charging mode is automatically selected and the battery remains in this state until the stabilized power supply unit 8 is disconnected from the electric mains or processing unit is switched off. High-rate charging is evidenced by illumination of a blinking sign on the indicator 11.
2. The stabilized power supply unit 8 is connected to the processing unit 6, which is not fitted with the storage battery 9.
   When the power is supplied the power supply unit 8 is connected to the electric mains, to the processing unit 6.
3. The stabilized power supply unit 8 is not connected to the processing unit 6, which encloses the storage battery 9. The processing unit 6 draws the electric power from the battery.
   The stabilized power supply unit 8 is made as a separate device or is built the processing unit 6. The electric mains and output wires are non-split. There is a light-emitting diode which indicates connection of the power supply unit 8 to the AC mains. The board installed inside the casing mounts a mains transformer, electronic components, mains and output fuses.

Structurally, the processing unit 6 is a compact table-mounted device. The casing of the processing unit 6 is made of shock-proof plastic and consists of two parts which are screwed together. The screw holes in the bottom part of the casing are covered with rubber feet attached by glue. The processing unit 6 comprises its own tactile keyboard 10. The board installed inside the unit casing mounts the electronic components.

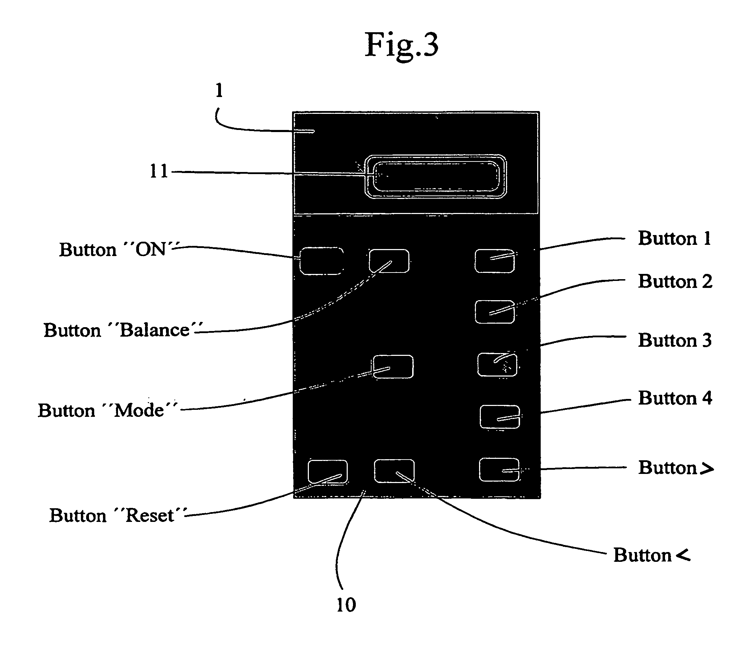

The layout of control and indicating organs on the processing unit 6 is shown in Fig. 3. For the display of sign information, the processing unit makes use of a two-line sign-synthesizing liquid-crystal indicator with light-emitting diode illumination.

The keyboard 10 of the processing unit 6 mounts the following buttons. (fig 3)
Button 1 serves for selection of the computer data processing program when measuring the potentials at the first combination of the biologically active points.
Button 2 serves for selection of the computer data processing program when measuring the potentials at the second combination of the biologically active points.
Button 3 serves for selection of the computer data processing program when measuring the potentials at the third combination of the biologically active points.
Button 4 serves for selection of the computer data processing program when measuring the potentials at the fourth combination of the biologically active points.

Button < serves for selection of the "Demonstration" mode and for generation of the pseudopotentials in the range of (0 - 100) mV at the increments of 1 mV.

When this button is depressed for the first time, the "Demonstration" mode is selected. When the button is repeatedly depressed (or is depressed and held in this position), the required value of the pseudopotential is generated.

Button > serves for selection of the "Demonstration" mode and for generation of the pseudopotentials in the range of (0 + 100) mV at the increments of 1 mV.

When this button is depressed for the first time, the "Demonstration" mode is selected. When the button is repeatedly depressed (or is depressed and held in this position), the required value of the pseudopotential is generated.

Button "Balance" is a multifunctional one. Besides, there are buttons marked "ON", "Mode", and "Reset".

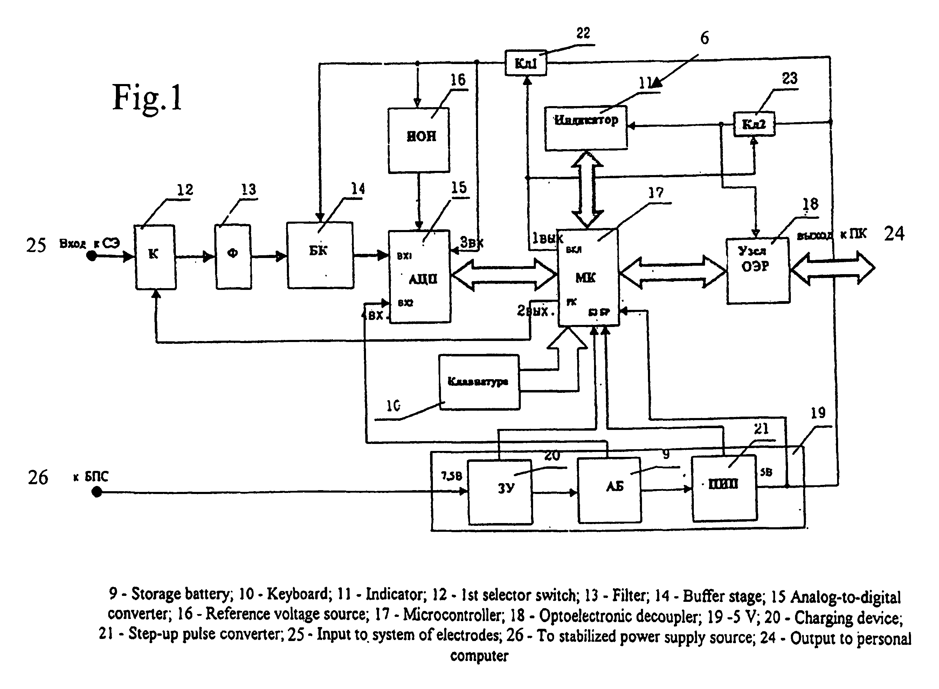

The processing unit 6 whose functional diagram is shown in Fig. 1 consists of the first selector switch 12, the filter 13, the buffer stage 14, the analog-to-digital converter 15 with the reference voltage source 16, the microcontroller 17, the indicator 11, the optoelectronic decoupler 18, the power supply unit 19 comprising the charging device 20, the step-up pulse converter 21, the storage battery 9, the second and third selector switches 22, 23 as well as the input connectors - the "Input" connector for coupling the electrodes 4, 5, the " 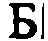 ΠC" (26) connector for coupling tie stabilized power supply unit 8, the "ΠK" (24) connector for coupling the personal computer 2, the cover of the "A 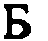 " (storage battery 9) compartment.

The bipolar voltage produced with the aid of the system 3 of electrodes is supplied to the input of the processing unit 6. The first selector switch 12 (e.g. the sealed-contact reed relay) shortens the input in the "Control" mode and is governed by the control command transmitted directly from the microcontroller 17. The third selector switch 22 is governed by another command transmitted from the microcontroller 17. The low-frequency filter 13 suppresses the differential and common-mode interference. From the output of the filter 13 the signal is fed to the input of the buffer stage 14 build around a micropower precision operational amplifier connected as a voltage repeater. The buffer stage 14 is intended for matching the input resistance of the processing unit 6 with the internal resistance of the source signal, which ensures the required sensitivity and accuracy of measurements. From the output of the buffer stage 15 the signal is fed 10 the analog-to-digital converter 15 which converts the analogous signal into a digital code.

The first (main) channel of the analog-to-digital converter is configured for operation in the bipolar mode The signal from the output of the buffer stage 14 is fed to its first analogous input - (BX.1). The second (auxiliary) channel of analog-to-digital converter 15 is configured for operation in the unipolar mode. The voltage from the storage battery 9 is supplied to its fourth analogous input (BX.2). The stabilized reference voltage required for operation of the analog-to-digital convener 15 is supplied from the reference voltage source 16. Thus, the first channel of the analog-to-digital converter is used for conversion of the input signal, and the second channel is used for checking the state of the battery 9.

From the output of the analog-to-digital converter 15 the digital code is fed through the successive interface to the microcontroller 17, which establishes the sequence of operation of the channels of the analog-to-digital convener 15;
readout of the results of conversions made by the analog-to-digital converter 15 in the form of the digital code;
transformation of the digital code supplied from the analog-to-digital converter 15 into the respective voltage, control of functioning of the indicator 11;
transmission of the measurement results through the successive interface to the personal computer 2 for their further processing;
transmission of the "I" and "4" control commands through the successive interface for selection of the respective data processing programs of the computer 2;
reception of the control command through the successive interface from the computer 2 for selection of the system balancing in the "Operation" mode or selection of the initial balancing in the "Demonstration" mode; control of the processing unit 6 with the aid of the keyboard;
generation of the pseudopotentials in the range of ±100 mV at the increments of 1 mV in the "Demonstration" mode;
Check of the minimum voltage of the storage battery 9, check of high-rate charging of the storage battery 9 by the "High-rate charging" ( 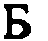 P) signal from the charging device 20;
power saving by the storage battery 9 when me time of the interval in the access (from the keyboard or by transmission of the control command from the personal computer 2) is over 25 min which initiates disconnection of electric power from the processing unit 6 (in this case, the microcontroller 17 is changed over into the low-consumption ("stand-by") condition.

The indicator 11 is a two-line sign-synthesizing L quid-crystal indicator with light-emitting diode used for illumination. The 8-digit data bus is used as a system interface.

The optoelectronic decoupler 18 ensures decoupling of the electric circuits of the processing unit 6 and personal computer 2.

The power supply unit 19 is made up of the charging device 20 and of the step-up pulse converter 21.

The charging device 20 is intended for charging the battery 9 and for supply of power to the step-up pulse converter 21 in the buffer condition.

The step-up pulse converter 21 is intended for conversion of the input non-stabilized voltage into the output-stabilized voltage, which is required for supply to the processing unit 6. The "Battery is discharged" signal is transmitted from the unit 21 to the microcontroller 17. After the transmission of this signal, the processing unit 6 is deenergized.

The electric mammograph operates as follows.

After the instrument is switched on within the computer complex, a blinking line reading "Instrument is switched on" and a green signal dot should be displayed in the lower right window on the screen of the monitor of the computer 2, and the digits corresponding to the readings of the liquid-crystal indicator 11 of the processing unit 6 should be displayed in the lower middle window. If these symbols are not displayed after switching on the processing unit 6, check proper connection of the processing unit 6 to the computer 2. Then, set the parameters of measurement. To this end, enter the "Setting" section of the main menu and then enter the "Configuration" subsection. The measurement parameter settings will be displayed in the "Configuration" dialogue window, which appears in the main "Measurement" field. For instance, the following values of the measurement parameters can be set:
- initial delay -2s;
- interval between measurements - 500 ms;
- permissible measurement limits - 90 mV;
- number of points - 30.

The measurement parameters which have been set are locked by the "OK" command. In the second field of the "Configuration" dialogue window, which is marked "Print", select a full or abridged format of the "Examination card". Then, enter the "Patient" menu and select the "New patient" line. As a result, he dialogue window marked "Information about patient" will be displayed on the screen. Introduce the information about the patient and number of the current examination file. After that, connect the system 3 of the electrodes to input connectors of the processing unit 6. In this case, the electrodes 4, 5 are dipped into the working solution. Further, make the system balancing, after, which proceed to taking measurement. To this end, place the electrodes in the biologically active points of the patients in conformity with the 1st combination.

After termination of the transient process, check the instrument readings in the indicator window and then strike key "1" in the upper line of the menu with the use of a mouse or depress button "1" on the processing unit 6. As a result, the coordinate grid and current realization of the process, which is measured, will be displayed in the working field of the "Diagnostic" window after an initial delay. The nominal value of the measurement in millivolts (mV) is read on the horizontal axis of the coordinate grid and the time scale is read on the vertical axis.

The time length of realization at the selected values of the measurement parameters is about 15s (30 points, with the intervals between the measurements equal to 0.5 s).

After completion of the 1st realization of the process which is measured, the following statistical data will be displayed in the supplementary window marked "Examination statistics":The mean value, the maximum amplitude of deviation from the mean value, the root-mean-square value (dispersion). The initial delay determines a time shift between the moment when the key (button) 1 is depressed and the moment when the pickup of the data from the processing unit 6 is commenced this delay can vary, depending on the examination conditions.

Then, place the electrodes in the patient's biologically active points according to the 2^{nd} combination, wait for termination of the transient process, and strike key "2" in the upper line of the menu with the aid of the mouse or depress the button *2* on the processing unit 6. After the completion of the second realization of the process, which is measured, its statistical data are automatically processed and the processing results are displayed in the "Examination statistics" window. If it becomes necessary to extract the non-peculiar points from the obtained realization, make the required correction of the sample. Make similar measurements for the 3rd and 4^{th} combinations. After completion of all four measurements (when all columns in the "Examination statistics" window are filled in with the measurement results), the "Examination results" dialogue window is automatically formed, which graphically shows the examination results and symptoms allowing for attribution of the patient to the "group of risk", while the result of the computer examination (summary) stated under the graphical picture can be expressed as follows: "Norm", "Group of risk". The symptoms allowing for attribution of the patient to the group of risk and the excess of the threshold value for each symptom are stated in brackets.

In the "Operation" mode, the difference of the potentials supplied to the input of the processing unit is measured. The measurement results are shown on the display of the processing unit 6 and are transmitted through the successive interface to the computer 2.

In the "Control" mode, the input of the processing unit 6 is automatically shorted and the input bias voltage of the instrument, i.e. its initial unbalance is measured. The "Control" mode is intended for initial balancing of the processing unit 6 and for self-test.
The "Demonstration" mode is selected by depression of the < or > buttons. In this case, the input measuring part of the processing unit 6 does not function. After one of these buttons is repeatedly depressed or it is depressed and held in position, the processing unit 6 generates the pseudopotentials in the range of ±100 mV at the increments, of 1 mV, which are displayed on the indicator 11 of the processing unit 6 and are simultaneously transmitted through the successive interface to the personal computer 2.

### Industrial applicability

The electric mammograph is intended for prompt examination (screening) of mammary glands by the electric mammography method with the view of detection of their pathology at the early stages of the disease. The apparatus can be used for conduct of examination in the hospital and ambulatory conditions and provides for examination of the mammary glands at home with a high degree of authenticity of the obtained results, which allows for detection of pathology, if any. If the electrical mammograph according to the invention is coupled to a personal computer, the apparatus makes it possible to check the dynamics of the pathologic process and its recovery and allows the physician to correct the treatment algorithm in reference to a particular patient. The electric mammograph is portable, relatively cheap, simple in use, and allows for detection of the pathologic states of mammary glands at the early stages of the disease.

## Claims

1. An electric mammograph (1) comprising: a system of electrodes (3, 4, 5), the system of electrodes being connected to an input of a processing unit (6) and to a power supply unit (19), wherein the processing unit (6) includes connected in series: a first selector switch (12), a filter (13) and an analog-to-digital converter (15) connected by a first group of inputs-outputs to a microcontroller (17); the output of the filter (12) is connected to a first input of the analog-to-digital converter; the first selector switch (12) is connected to the output of the system of electrodes (3, 4, 5); the microcontroller (17) is connected by a second group of inputs-outputs to an optoelectronic decoupler (18); the output of a reference voltage source (16) is connected to a second input of the analog-to-digital converter (15); the input of the reference voltage source (16) is connected: to a second input of the buffer stage (14), to the output of a second selector switch (22) and to a third input of the analog-to-digital converter (15); the first output of the microcontroller (17) is connected to the second and a third selector switches (22, 23); a second output of the microcontroller (17) is connected to the first selector switch (12); inputs of the microcontroller are connected to the power supply unit (19); a fourth input of the analog-to-digital converter (15) is connected to a storage battery (9); the output of the third selector switch (23) is connected to an indicator (11) connected by a group of inputs-outputs to the microcontroller (17) and to the optoelectronic decoupler (18).

2. An electric mammograph according to Claim 1, **characterized in that** the power supply unit (19) comprises connected in series a charging device (20) and a step-up pulse converter (21), which are connected to the microcontroller (17) inputs.

3. An electric mammograph according to Claim 1 or 2, **characterized in that** a two-line sign-synthesizing liquid-crystal indicator with the light-emitting diode illumination is used as an indicator (11).

4. An electric mammograph according to Claims 1 or 2 or 3, **characterized in that** provision is made for selection of a "Demonstration" mode.

5. An electric mammograph according to any of the foregoing Claims, **characterized in that** the microcontroller (17) is connected by a group of inputs-outputs to its own keyboard (10) for selection of the functional modes.

## Patentansprüche

1. Elektrischer Mammograph (1), enthaltend: ein System von Elektroden (3, 4, 5), wobei das System der Elektroden an den Eingang einer Prozessoreinheit (6) und an eine Energieversorgungseinheit (19) angeschlossen ist, wobei die Prozessoreinheit (6) in Reihenschaltung aufweist: einen ersten Wahlschalter (12), ein Filter (13) und einen Analog-Digital-Umwandler (15), der über eine erste Gruppe von Eingängen und Ausgängen mit einem Mikrokontroller (17) verbunden und der Ausgang des Filters (12) an einen ersten Eingang des Analog-Digital-Umwandlers gelegt ist; wobei der erste Wahlschalter (12) mit dem Ausgang des Systems der Elektroden (3, 4, 5) verbunden ist; der Mikrokontroller (17) über eine zweite Gruppe von Eingängen und Ausgängen an einen optoelektronischen Entkoppler (18) geschaltet ist; der Ausgang einer Bezugsspannungsquelle (16) an einen zweiten Eingang des Analog-Digital-Umwandlers (15) gelegt ist; der Eingang der Bezugsspannungsquelle (16) mit einem zweiten Eingang der Pufferstufe (14), mit dem Ausgang eines zweiten Wahlschalters (22) und einem dritten Eingang des Analog-Digital-Umwandlers (15) verbunden ist; der erste Ausgang des Mikrokontrollers (17) an den zweiten und einen dritten Wahlschalter (22, 23) gelegt ist; ein zweiter Ausgang des Mikrokontrollers (17) an den ersten Wahlschalter (12) geschaltet ist; Eingänge des Mikrokontrollers an der Energieversorgungseinheit (19) liegen; ein vierter Eingang des Analog-Digital-Umwandlers (15) mit einer Speicherbatterie (9) verbunden ist; der Ausgang des dritten Wahlschalters (23) an eine Anzeige (11) gelegt ist, welche über eine Gruppe von Eingängen und Ausgängen mit dem Mikrokontroller (17) und dem optoelektronischen Entkoppler (18) verbunden ist.

2. Elektrischer Mammograph nach Anspruch 1, **dadurch gekennzeichnet, dass** die Energieversorgungseinheit (19) eine Reihenschaltung einer Ladevorrichtung (20) und eines Aufwärts-Impulswandlers (21) enthält, welche mit Eingängen des Mikrokontrollers (17) verbunden ist.

3. Elektrischer Mammograph nach Anspruch 1 oder 2, **dadurch gekennzeichnet, dass** als Anzeige ein zweikanalzeichensynthetisierender Flüssigkristall-Indikator mit Leuchtdiodenbeleuchtung (11) verwendet ist.

4. Elektrischer Mammograph nach Anspruch 1 oder 2 oder 3, **dadurch gekennzeichnet, dass** Vorkehrungen zur Auswahl eines "Demonstrations"-Modus getroffen sind.

5. Elektrischer Mammograph nach einem der vorstehenden Ansprüche, **dadurch gekennzeichnet, dass** der Mikrokontroller (17) über eine Gruppe von Eingängen und Ausgängen mit einer eigenen Tastatur (10) zur Auswahl der Funktionsmodi verbunden ist.

## Revendications

1. Mammographe électrique (1) comprenant : un système d'électrodes (3, 4, 5), ce système étant relié à une entrée d'une unité de traitement (6) et à une unité d'alimentation en énergie (19), dans lequel l'unité de traitement (6) comprend, connectés en série, un premier commutateur de sélection (12), un filtre (13) et un convertisseur analogique-numérique (15) relié via un premier groupe d'entrées et de sorties à un microcontrôleur (17); la sortie du filtre (12) est connectée à une première entrée du convertisseur analogique-numérique; le premier commutateur de sélection (12) est relié à la sortie du système d'électrodes (3, 4, 5); le microcontrôleur (17) est connecté via un deuxième groupe d'entrées et de sorties à un découpleur optoélectronique (18); la sortie d'une source de tension de référence (16) est reliée à une deuxième entrée du convertisseur analogique-numérique (15); l'entrée de la source de tension de référence (16) est connectée à une deuxième entrée d'un étage séparateur (14), à la sortie du deuxième commutateur de sélection (22) et à une troisième entrée du convertisseur analogique-numérique (15); la première sortie du micro-contrôleur (17) est connectée au deuxième et à un troisième commutateur de sélection (22, 23); une deuxième sortie du microcontrôleur (17) est reliée au premier commutateur de sélection (12); des entrées du microcontrôleur sont connectées à l'unité d'alimentation en énergie (19); une quatrième entrée du convertisseur analogique-numérique (15) est reliée à une batterie secondaire (9); la sortie du troisième commutateur de sélection (23) est connectée à un indicateur (11) relié via un groupe d'entrées et de sorties au microcontrôleur (17), et au découpleur optoélectronique (18).

2. Mammographe électrique selon la revendication 1, **caractérisé en ce que** l'unité d'alimentation en énergie (19) comprend, reliés en série, un dispositif de charge (20) et un survolteur à impulsions (21), dispositifs qui sont reliés aux entrées du micro-contrôleur (17).

3. Mammographe électrique selon la revendication 1 ou 2, **caractérisé en ce qu'**un indicateur à cristaux liquides synthétisant des signes de deux lignes et doté d'une illumination par diodes luminescentes est utilisé comme indicateur.

4. Mammographe électrique selon la revendication 1 ou 2 ou 3, **caractérisé en ce que** des dispositions sont prévues pour choisir un mode de "démonstration".

5. Mammographe électrique selon l'une quelconque des revendications précédentes, **caractérisé en ce que** le micro-contrôleur (17) est relié via un groupe d'entrées et de sorties à son propre clavier (10) pour une sélection des modes fonctionnels.
